# EUROPEAN PATENT APPLICATION

(11) **EP 4 706 557 A1**
(43) Date of publication of application: **11.03.2026**
(21) Application number: 25200094.8
(22) Date of filing: 04.09.2025
(51) Int. Cl.: A61B 17/16

(54) **LEVER ADJUSTMENT MECHANISM FOR SURGICAL TOOL ACCESSORY**

(30) Priority: 05.09.2024 US 202463691056 P; 29.08.2025 US 202519314071
(71) Applicant: Arthrex, Inc, Naples, FL 34108-1945 (US)
(72) Inventor: Twomey, John Ryan, Naples, FL, 34108 (US); Lombardo, Giuseppe, Naples, FL, 34108 (US)
(74) Representative: Lohr, Jöstingmeier & Partner Patent- und Rechtsanwälte mbB

(57) **Abstract**

A method and apparatus for adjusting an orientation of a lever arm of a collet assembly of a rotary surgical tool includes determining a travel or throw of the lever arm of an actuator lever of the collet assembly relative to a handle of the rotary surgical tool in one of a clamped position and a released position. The method further includes adjusting an orientation of a pivot pin of the apparatus about a pin axis and defining a lever axis about which the lever arm rotates. The orientation of the pivot pin relative to the collet assembly defines an offset direction of the lever axis relative to the pin axis.

## Description

### CROSS-REFERENCE TO RELATED APPLICATIONS

This application claims priority under 35 U.S.C. § 119(e) and the benefit of U.S. Provisional Application No. 63/691,056 entitled LEVER ADJUSTMENT MECHANISM FOR SURGICAL TOOL ACCESSORY, filed on September 5, 2024, by John Ryan Twomey, et al., the entire disclosure of which is incorporated herein by reference.

### BACKGROUND

The present disclosure generally relates to an actuation mechanism for selectively clamping or gripping pins or dowels for mechanical manipulation. More particularly, the disclosure relates to an adjustment mechanism for an actuation or lever arm for a gripping mechanism used to engage pins or wires for surgical applications.

### SUMMARY

Surgical accessories and tools may be applied to support a wide variety of procedures and surgical operations. In various operations, wires, pins, dowels, and/or various elongated support structures may be utilized to provide alignment or structural support. The disclosure provides for an actuator assembly for a collet of a surgical tool that may be configured to selectively grip or engage pins or wires associated with surgical procedures. The selective engagement or clamping of the collet may be controlled based on an orientation of a lever relative to a handle of a rotary power tool. In this configuration, the lever may rotate about a lever axis or fulcrum to clamp and unclamp the collet and lock the pin or wire in connection with a rotary drive of the power tool. In addition to clamping the elongated body of the wire or pin, the clamping of the collet may align the elongated body with a longitudinal axis defining a drive path and trajectory of the pin or wire. In various implementations, the disclosure may provide for an adjustment assembly including an indexing locator that may adjust a position of the lever axis, thereby adjusting an orientation of the lever in both a clamped and unclamped position. In this way, the disclosed actuator assembly may be adjusted to ensure the lever is positioned conveniently and ergonomically relative to the handle of the surgical rotary power tool.

In various implementations, the actuator assembly may be incorporated in a driver attachment or pin driver attachment that may be interchangeably connected to a rotary power tool. In such implementations, the indexing locator may engage a support bracket of a body of the driver attachment. As further discussed in reference to various exemplary implementations, the body of the driver may correspond to a structural housing that may operably couple a drive interface to a drive assembly of the rotary power tool and further house the collet of the actuator assembly. In such implementations, the indexing locator may be selectively positioned at one of a plurality of rotational positions relative to the support bracket, thereby defining the position of the lever axis of the lever. The resulting position of the lever axis may adjust the orientation of the lever relative to the actuator assembly and the handle of the power tool. Accordingly, the disclosure may provide for effective adjustment of the orientation of the actuator assembly to correct for various tolerances or manufacturing variations associated with the driver attachment.

These and other features, objects and advantages of the present disclosure will become apparent upon reading the following description thereof together with reference to the accompanying drawings.

### BRIEF DESCRIPTION OF THE DRAWINGS

FIG. 1 is a partially exploded assembly view demonstrating a driver attachment and associated connection interface of a power tool;
FIG. 2 is a partially exploded assembly view demonstrating primary subassemblies of a driver attachment for a surgical power tool;
FIG. 3A is a front cross-sectional assembly view, sectioned along line A-A in FIG. 1, demonstrating a pivot pin of an actuator assembly;
FIG. 3B is a projected isometric view of a pivot pin of an actuator assembly for a pin or wire driver;
FIG. 4A is a side cross-sectional assembly view of the driver attachment along section line B-B demonstrated in FIG. 3A;
FIG. 4B is a detailed sectional view of a pivot pin demonstrating an indexing locator;
FIG. 5 is an exploded assembly view demonstrating a driveshaft assembly of a wire or pin driver attachment for a surgical power tool;
FIG. 6A is a side profile view of a driveshaft assembly of a wire or pin driver attachment for a surgical power tool;
FIG. 6B is a transverse section view along section line C-C demonstrated in FIG. 6A of the driveshaft assembly demonstrating a ball lock interface of a collet;
FIG. 7 is a detailed sagittal section view of a driveshaft assembly including a detailed view D of a ball lock interface;
FIG. 8A is a detailed sagittal section view of the driveshaft assembly demonstrating the ball lock interface with an actuation lever in an open state; and
FIG. 8B is a detailed sagittal section view of the driveshaft assembly demonstrating the ball lock interface with an actuation lever in a closed state.

### DETAILED DESCRIPTION

In the following description, reference is made to the accompanying drawings, which show specific implementations that may be practiced. Wherever possible, the same reference numbers will be used throughout the drawings to refer to the same or like parts. It is to be understood that other implementations may be utilized and structural and functional changes may be made without departing from the scope of this disclosure.

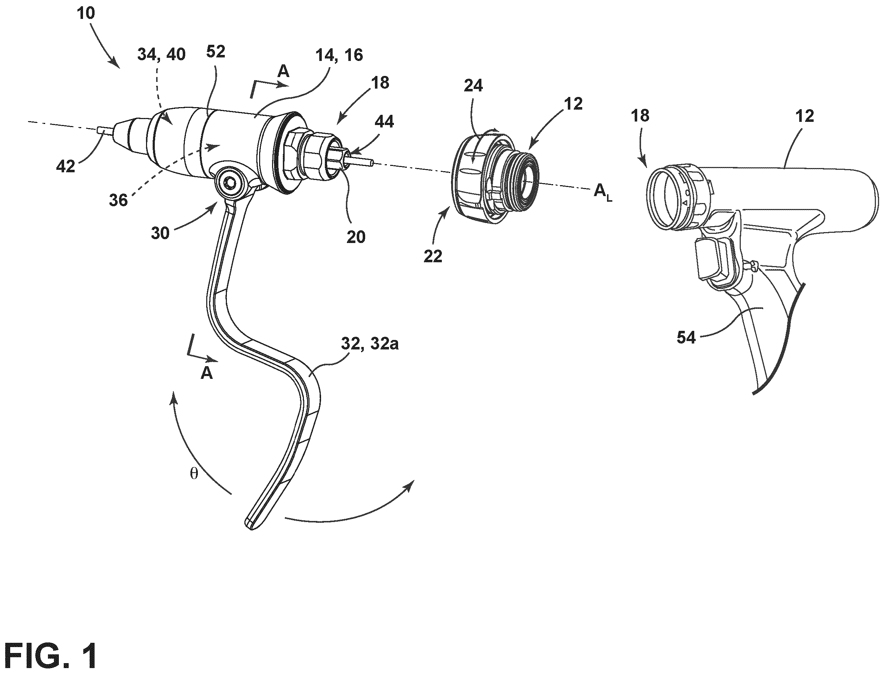

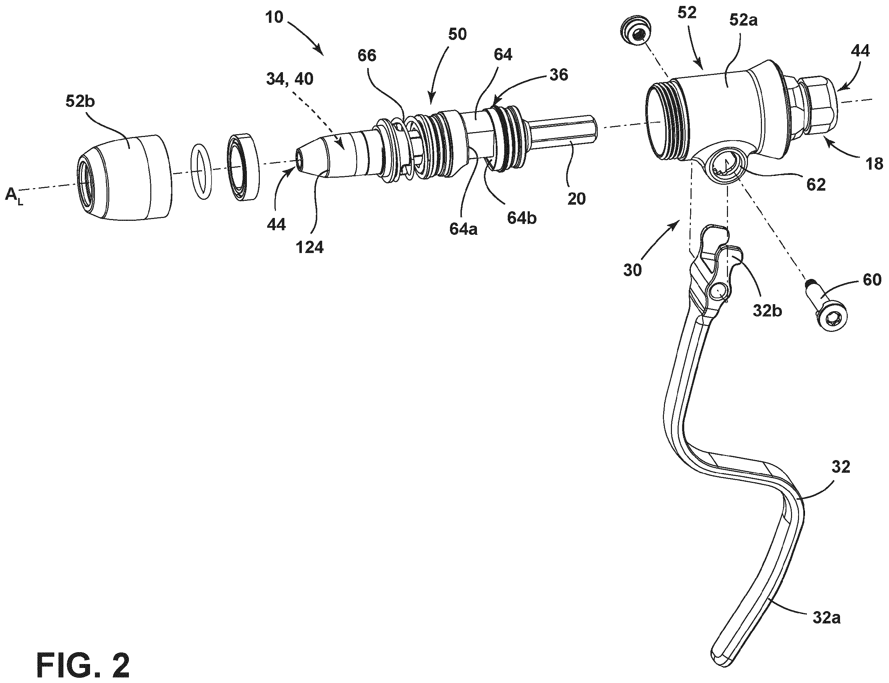

Referring generally to FIGS. 1 and 2, the disclosure provides for the improved operation of a pin or wire driver or driver assembly 10 for a rotary power tool 12. Though demonstrated as an interchangeable tool accessory 14, the disclosure may generally be relevant to the operation of various rotary surgical tools that may provide for the selective clamping or engagement of objects with collets or other similar clamping mechanisms. As illustrated in FIG. 1, the driver assembly 10 may correspond to a drive head 16 including a drive interface 18 comprising a driveshaft 20. In this configuration, the drive interface 18 may be selectively engaged with a receiving coupler 22 operably coupling the driver assembly 10 to the rotary power tool 12. The engagement and disengagement of the drive head 16 may be locked and released via a rotating collar 24, allowing the driver assembly 10 to be alternatively utilized with a variety of interchangeable tool accessories 14.

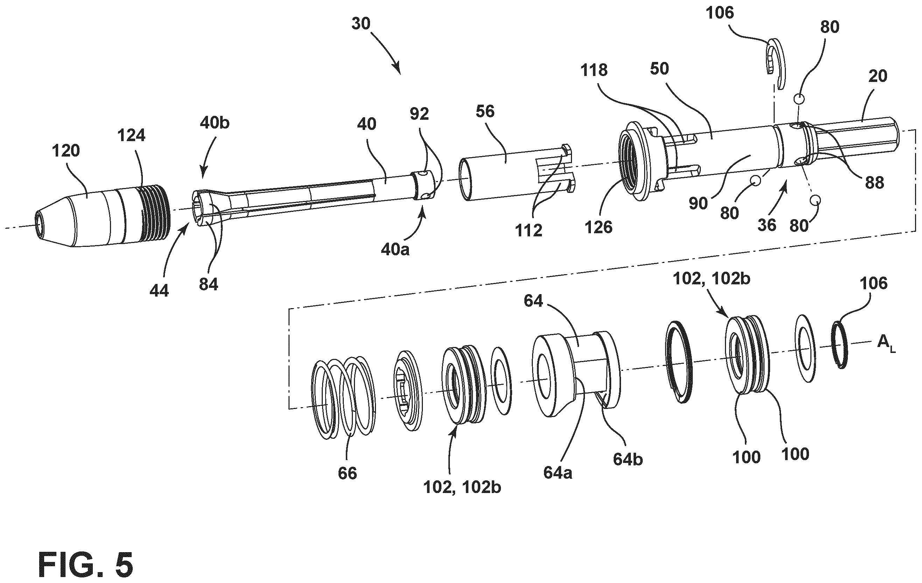

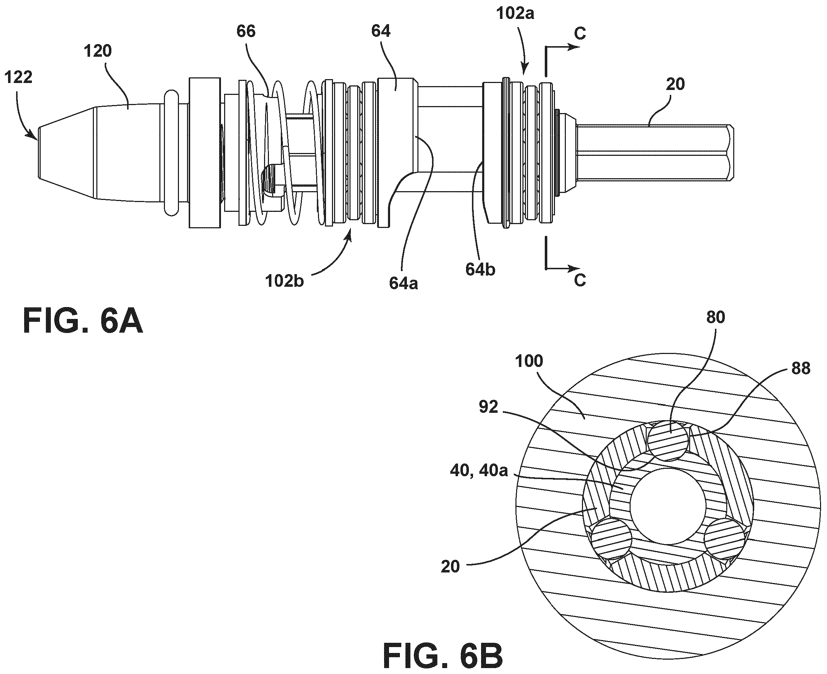

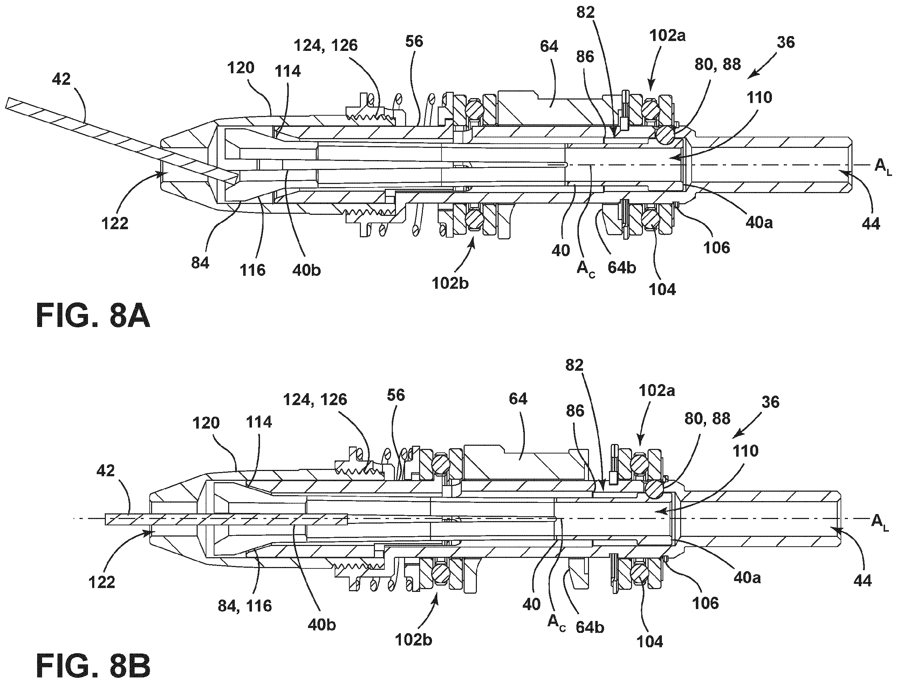

As demonstrated in the following detailed examples, the disclosure may provide for two operably related features that may improve the operation of the driver assembly 10. As discussed primarily in reference to FIGS. 2, 3A, 3B, 4A, and 4B, an actuator assembly 30 is disclosed that may provide for the effective adjustment of an angular orientation or lever angle θ of a lever 32 configured to engage and disengage a clamping or contraction of a collet assembly 34 operably connected to the driveshaft 20. Further, as described in reference to FIGS. 5, 6A, 6B, 7, 8A, and 8B, the disclosure may provide for various features related to a locking interface 36 that may flexibly couple the collet assembly 34 to the driveshaft 20. In operation, the locking interface 36 may be engaged and disengaged by adjusting the lever angle θ with the lever 32, thereby causing the actuator assembly 30 to clamp or contract a collet to enclose about a wire, pin 42, or similar elongated insert. Accordingly, the collet assembly 34 may provide for the selective engagement of the wire or pin 42 extending through a cannula 44 of the driver assembly 10 and the driveshaft 20 (see FIGS. 8A and 8B). The effective clamping of the collet assembly 34 may provide for improved operation of the driver attachment for various applications.

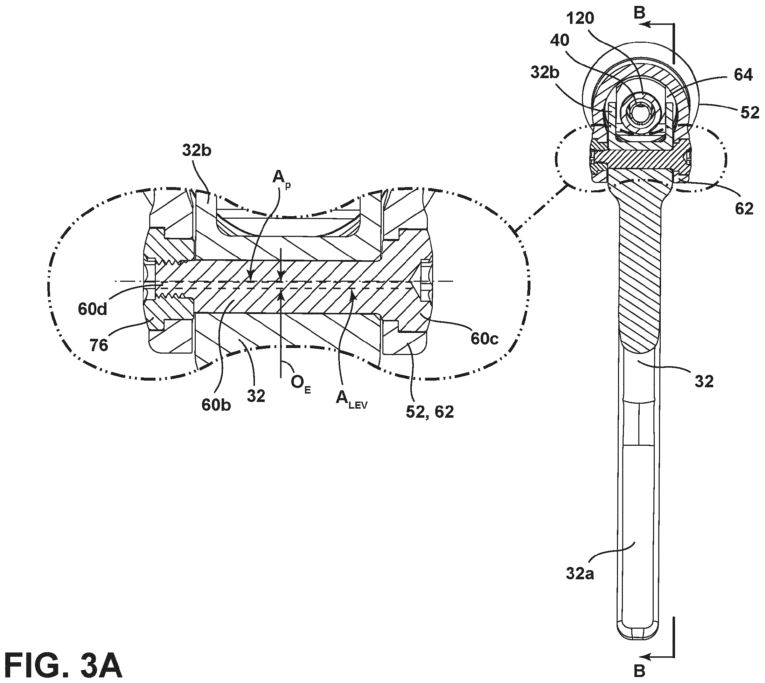

Still referring to FIGS. 1 and 2, the driver assembly 10 may generally comprise a plurality of subassemblies including a driveshaft assembly 50 that may be housed within a body assembly 52 and engaged by the lever 32 to engage and release the collet assembly 34. The subassemblies 34, 50, and 52 are generally demonstrated in the exploded view shown in FIG. 2 and demonstrated in further detail in FIGS. 3A-8B. As may be apparent in FIGS. 1 and 2, in the assembled configuration, the driver assembly 10 may engage the rotary power tool 12, such that a force arm 32a of the lever 32 extends along a handle 54 of the rotary power tool 12. In this configuration, an operator of the tool 12 may clamp and unclamp the collet 40 by adjusting the force arm 32a of the actuator assembly 30 to translate a closing collar or closer 56 (see FIG. 5). In the unclamped configuration, the collet 40 may be free to dilate or open to receive the pin 42. In the clamped configuration, the closer 56 may enclose about and contract the collet 40, locking the pin 42 within the collet 40. For example, an operator may adjust the actuator assembly 30 into a clamped position by adjusting the force arm 32a to reposition an opposing load arm 32b of the lever 32 in a counterclockwise or clockwise direction as demonstrated by the annotated arrows in FIG. 1. In this configuration, the lever angle θ of the lever 32 relative to the body assembly 52 can be moved in varying proximity with the handle 54 of the tool 12. Accordingly, the position of the lever 32 in both the clamped and unclamped positions may be important to ensure the user can easily reach and engage the force arm 32a of the lever 32 while holding the handle 54 of the tool 12.

As best demonstrated in FIG. 2 and further described in reference to FIG. 5, the collet assembly 34 may correspond to a subassembly or component of the overall driveshaft assembly 50 of the driver 10. For clarity, the actuator assembly is first described in reference to FIGS. 2-4 and additional features of the collet assembly 34 and driveshaft assembly 50 are discussed later in reference to FIGS. 5-8. As shown in FIG. 2, a load arm 32b of the lever 32 may extend into a proximal body portion 52a of a body assembly 52 or housing of the driver assembly 10. In this configuration, the lever 32 may engage the body assembly 52 via a pivot pin 60 connected to a support bracket 62 in connection with or formed by the proximal body portion 52a. In operation, the rotation of the lever 32 about the pivot pin 60 may cause the load arm 32b to apply force to a clamping surface 64a and a releasing surface 64b of a pusher 64 in response to a clockwise or counterclockwise rotation.

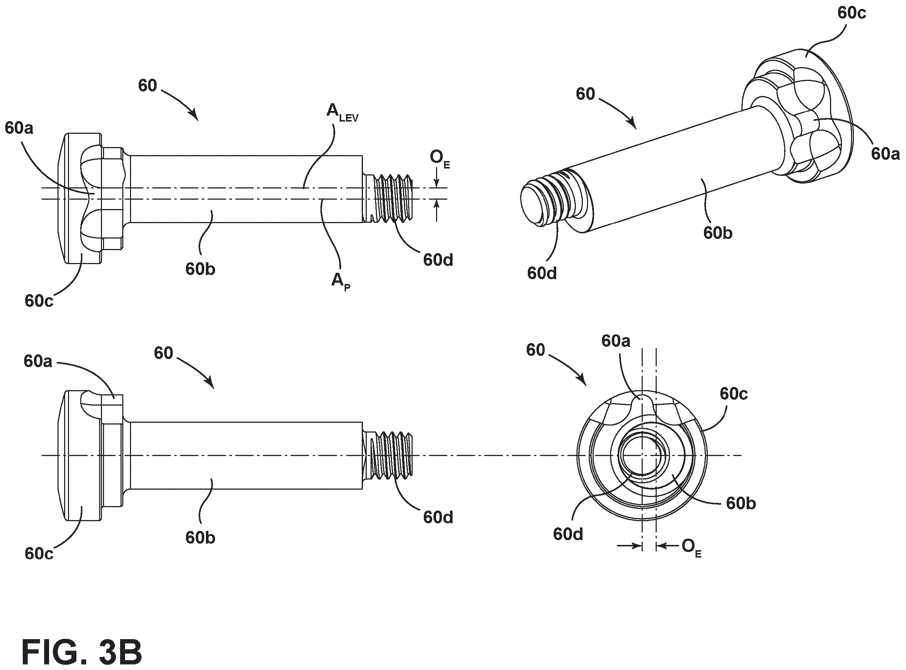

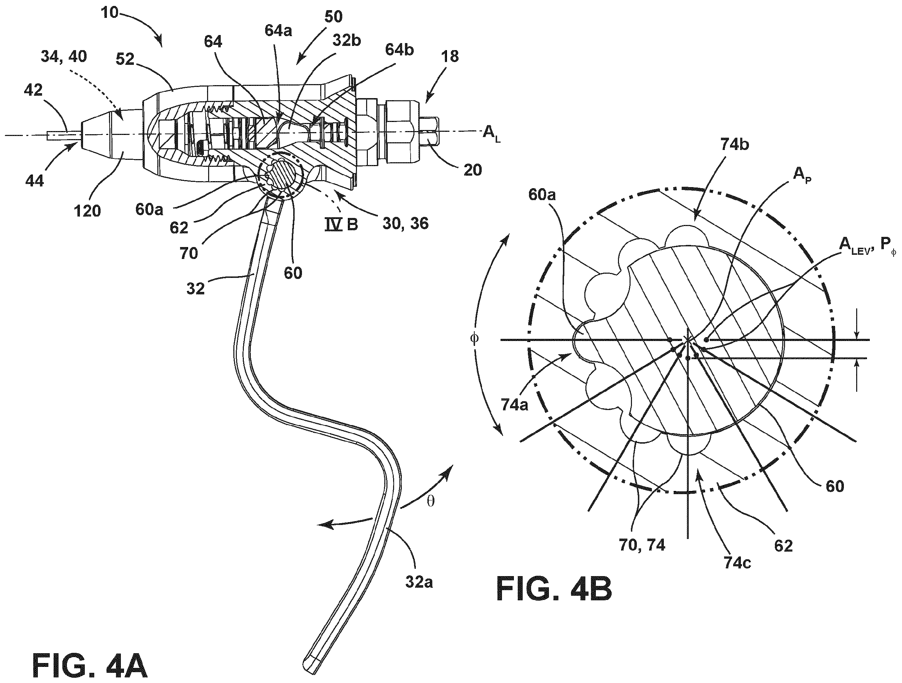

As shown, the pusher 64 may correspond to a collar that is enclosed about the driveshaft 20. In operation, the pusher 64 may apply an axial force along a longitudinal axis A_{L} to the closing collar 56 to clamp and release the collet 40. In addition to the lever 32, the release of the pusher 64 and the dilation of the collet 40 may be assisted by a compression or release spring 66 that may apply opposing force to that applied by the load arm 32b of the lever. As shown, the components of the collet assembly 34 and, more generally, the driveshaft assembly 50 shown in FIG. 5, may include several mating parts that may be subject to manufacturing variations in the position of the pusher 64 relative to the pivot pin 60. As further described in detailed reference to FIGS. 3 and 4, a pin orientation ϕ of the pivot pin 60 may adjust a rotational position P_{ϕ} of an indexing locator 60a and a resulting position of a lever axis A_{LEV} about a pivot pin axis or pin axis A_{P}. In this way, a position of the lever axis A_{LEV} may be adjusted relative to the support bracket 62 to adjust the lever angle θ of the lever 32 when engaging the pusher 64 and the closer 56 or closing collar allowing the collet to expand or causing the closure of the collet 40.

Referring now to FIGS. 3A, 3B, 4A, and 4B, the pivot pin 60 and support bracket 62 are further discussed in reference to the operation of the indexing locator 60a. As shown, the indexing locator 60a may be selectively positioned in one of a plurality of guide slots 70 formed by the support bracket 62. In this way, the lever axis A_{LEV} may be adjusted based on an orientation of the pivot pin 60. As shown in FIG. 3A, the pin 60 may comprise an elongated body 60b extending between a proximal head 60c and a distal fastening portion 60d. At the head 60c and the fastening portion 60d, the pivot pin 60 may extend along the pin axis A_{P}. However, the elongated body 60b may extend along an eccentric axis aligned with the lever axis A_{LEV} offset by an eccentric offset O_{E}. Accordingly, while the elongated body 60b may correspond to a uniform or generally cylindrical shape, the longitudinal axis of the cylindrical form may extend along the lever axis A_{LEV} offset from the pin axis A_{P}. In this configuration, orientation of the indexing locator 60a (shown in FIG. 4B), may adjust a position of the lever axis A_{LEV} relative to the pusher 64 of the actuator assembly 30. In this way, a pin orientation Φ may adjust the position of the lever axis A_{LEV} relative to the support bracket 62 and the body assembly 52, thereby adjusting a resulting position of the lever angle θ in both the clamped and released positions over a throw 72 or clamping range of the force arm 32a.

As further shown in FIG. 3A, the lever axis A_{LEV} is laterally offset from the pin axis A_{P} over the eccentric offset O_{E}. In the example shown in FIG. 4B, the indexing locator 60a is located in a neutral position 74a. As further demonstrated and discussed in reference to FIGS. 4A and 4B, adjusting the orientation of the indexing locator 60a in the guide slots 70 or openings formed by the support bracket 62 may reposition the lever axis A_{LEV} ranging from a fore position 74b to an aft position 74c defining an adjustment range from the neutral position 74a. For example, the indexing locator 60a may be positioned in a plurality of predefined rotary positions 74 about the pin axis A_{P}. Among the plurality of rotary positions 74, the fore position 74b of the lever axis A_{LEV} may be achieved by positioning the indexing locator 60a in the furthest clockwise located position of the rotary positions 74. Alternatively, an aft position 74c of the lever axis A_{LEV} may be achieved by positioning the indexing locator 60a in the furthest counterclockwise located rotary position 74. Between the fore position 74b and aft position 74c, a plurality of positions, including the neutral position 74a, may adjust the lever axis A_{LEV} incrementally about the support bracket 62. The orientation of the pivot pin 60 associated with the position of the indexing locator 60a may be selected to adjust the lever angles θ associated with the actuator assembly 30 in the clamped position and the release position. In this way, the throw 72 of the force arm 32a of the lever 32 may be adjusted relative to the handle 54 of the tool 12.

Though described in reference to the clockwise and counter or anticlockwise rotations, it shall be understood that these rotational directions are dependent on perspective of the driver 10 and the lever 32 relative to the user. Accordingly, the rotary position 74 may generally provide a corresponding repositioning of the lever axis A_{LEV} and the resulting adjustment of the load arm 32b relative to the pusher 64. Further, it shall be understood that the orientation of the eccentric offset O_{E} may similarly be adjusted to be various rotary assemblies (a gear and complementary receiving pocket, a keyed shaft, etc.) to provide for similar selective alignment of the orientation or rotational position P_{ϕ} of the pivot pin 60 relative to the body assembly 52 and the pusher 64. Accordingly, the relative position of the throw 72 of the lever 32 relative to the handle 54 may be adjusted in various ways as provided by the disclosure.

Referring now to FIGS. 3B and 4B, the features of the pivot pin 60 are discussed in further detail. As previously described, the indexing locator 60a may be rotated about the pin axis A_{P} to selectively engage the corresponding guide slots 70 or support openings formed by the support bracket 62 or a bracket assembly. In various implementations, the indexing locator 60a may correspond to a protrusion formed between the proximal head 60c and the elongated body 60b. In this configuration, the indexing locator 60a may be hidden from view by an enlarged proportion of the proximal head 60c relative to the extent of the protrusion forming the indexing locator 60a. Once positioned within the complementary opening of the guide slot 70 formed by the support bracket 62, the pivot pin 60 may be secured to the proximal body portion 52a of the body assembly 52 via a mating fastener 76 configured to engage the distal fastening end 60d of the pivot pin 60. In this configuration, the pivot pin 60 may be retained within the support bracket 62 with the indexing locator 60a oriented in the selected rotary position 74.

As previously discussed, in addition to the protrusion depicted, the indexing locator 60a may correspond to an indentation or series of indentations or openings that may be configured to receive protrusions or alignment detents or pins associated with the support bracket 62. In other words, the mating engagement between the indexing locator 60a and the support bracket 62, demonstrated in the example shown, may be reversed and/or adapted in shape and form while maintaining the same operable coupling provided by the indexing locator 60a. It shall be understood that variations in the specific geometry between the pivot pin 60 and the support bracket 62 may be implemented in a variety of ways to provide the selected positioning of the pivot pin 60 in the plurality of rotary positions 74 to adjust the location of the lever axis A_{LEV} relative to support bracket 62 pivot axis of the proximal body portion 52a. In this way, the position of the load arm 32b relative to the pusher 64 may be adjusted to reposition the lever 32 relative to the handle 54 or body assembly 52. Accordingly, the instruction of the disclosure may be applied to provide for several variations of the pivot pin 60 and indexing locator 60a.

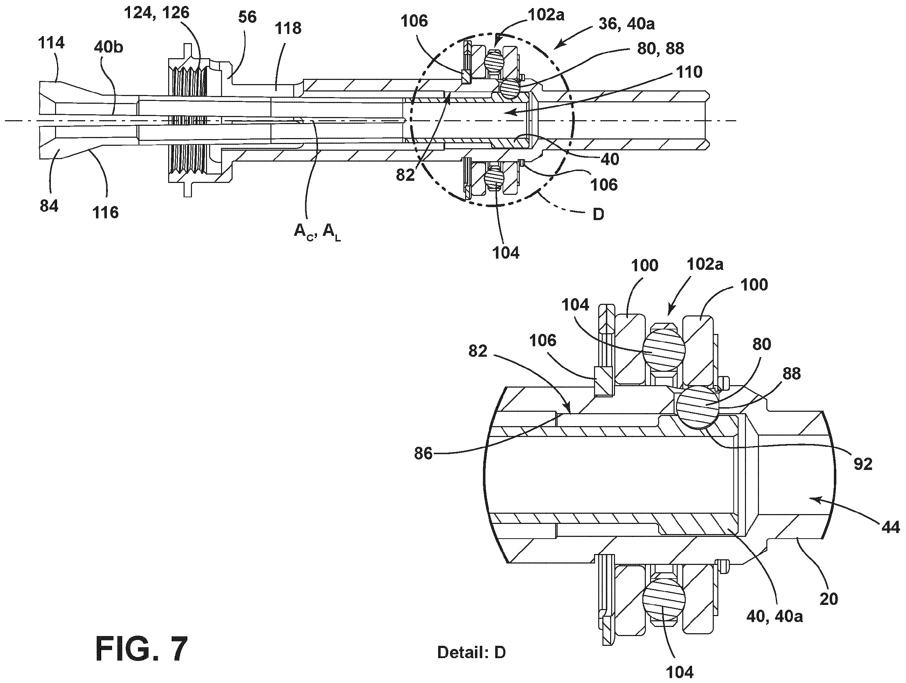

Referring now to FIGS. 5-8, the operation of the actuator assembly 30 is further discussed in reference to the engagement between the collet 40 and the driveshaft 20. As previously discussed, the actuation of the lever 32, may selectively clamp and release the collet assembly 34 to engage and disengage the locking interface 36 to align the collet 40 and the pin 42 with the longitudinal axis A_{L}. As best demonstrated in FIG. 5, the locking interface 36 may comprise at least one retaining insert 80 that may operably couple a proximal engagement portion 40a within a bore 82 (see FIG. 7) formed in the driveshaft 20. More specifically, the collet 40 may extend within the bore 82 from the proximal engagement portion 40a to a distal gripping portion 40b formed by a plurality of clamping arms 84. As best shown in FIG. 7, the bore 82 may extend from the cannula 44 formed through the driveshaft 20 and form a stepped wall 86 based on the increased proportion of the interior diameter of the bore 82 relative to the cannula 44. Once positioned in the bore 82 proximate the stepped wall 86, the proximal engagement portion 40a of the collet 40 may receive the at least one retaining insert 80, exemplified as a locking ball or sphere, through a locking aperture 88 or opening formed in a driveshaft wall 90 of the driveshaft 20. The retaining insert 80 may be received into a pocket 92 formed in a cylindrical exterior surface of the proximal engagement portion 40a of the collet 40. In this configuration, the retaining insert 80 may extend through the locking aperture 88 in the shaft wall 90 and into the pocket 92, thereby coupling the collet 40 within the bore 82 formed through the driveshaft 20.

Still referring generally to FIGS. 5-8 and particularly to FIG. 7, the retaining insert 80 may be maintained within the locking aperture 88 formed through the shaft wall 90 of the driveshaft 20 via a retaining ring 100. In the example shown, the retaining ring 100 may correspond to a portion of a bearing assembly 102 comprising a plurality of balls 104 or bearing balls, which may correspond to a proximal thrust bearing of the driveshaft assembly 50. In the example shown, the retaining ring 100 may correspond to a proximal washer of the bearing assembly 102 that may be held in position by axial positioning rings 106 in connection with the driveshaft 20 on opposing sides of the bearing assembly 102. In this configuration, the at least one retaining insert 80 may be confined within the locating aperture 88 and the pocket 92, thereby operably coupling the collet 40 within the bore 82 of the driveshaft 20 to form the locking interface 36.

As previously discussed, the locking interface 36 may allow the distal gripping portion 40b of the collet 40 to pivot within the bore 82. As demonstrated in FIGS. 8A and 8B, a pivot point 110, or pivot region, may be defined by the engagement of the proximal engagement portion 40a of the collet 40 and the at least one retaining insert 80 within the bore 82. The pivot point 110 or region may vary, in part based on the number of retaining inserts 80 forming the locking interface 36 about the driveshaft 20 and the proximal engagement portion 40a of the collet 40. Further, the spacing and clearance among the interior diameter of the bore 82 and the outside diameter of the proximal engagement portion 40a, as well as the relative proportions of the one or more retaining inserts 80 relative to the locking aperture(s) 88 and pocket(s) 92, may vary to provide a clearance for the collet 40 to pivot or shift within the bore 82. Generally, the proportions of the foregoing components forming the locking interface 36 may provide for adequate clearance, allowing the collet axis A_{C} to pivot within the bore 82 relative to the longitudinal axis A_{L} of the driveshaft 20, such that the distal gripping portion 40b of the collet 40 may be free to shift within the confines formed by an interior wall or the bore 82. In addition to the previously mentioned features of the locking interface 36, the proximal engagement portion 40a of the collet 40 may have a stepped or increased diameter relative to an elongated body 40c of collet 40 extending from the proximal engagement portion 40a to the distal gripping portion 40b formed by the clamping arms 84. In this configuration, the distal gripping portion 40b of the collet 40 may be free to move within a cone-shaped region defined by the variation of the collet axis A_{C} relative to the longitudinal axis A_{L} and extending from the pivot point 110 or pivot region.

As demonstrated, the locking interface 36 may be manufactured from components conducive to reliable and repeatable manufacture and assembly. For example, the retaining inserts 80 of the locking interface 36 is implemented as a spherical insert that may correspond to a ball bearing ball. Further, the locking aperture(s) 88, pocket(s) 92, and the interior diameter of the bore 82 may be manufactured via a variety of manufacturing techniques resulting in high precision without requiring specialty tooling or equipment (e.g., via drilling, boring, reaming, milling, etc.). In some cases, commonly available components (e.g., bearings) may be implemented for the inserts 80, such that standard, high-volume and precision components may be utilized to manufacture the collet assembly 34 while limiting manufacturing and assembly cost as well as waste from potential non-conforming parts. Accordingly, the collet assembly may utilize several standard components implemented in new ways to form the locking interface 36 to improve manufacturability.

The operation of the locking interface 36 may be related to additional components of the driveshaft assembly 50, which are now described in further detail. As discussed in reference to the lever 32 of the actuator assembly 30, the pusher 64 may be engaged by the load arm 32b of the lever on the clamping surface 64a and the releasing surface 64b. As demonstrated in FIGS. 8A and 8B, the pusher 64 is positioned in a released position and a clamped position, respectively. In operation, when force is applied to the clamping surface 64a by the load arm 32b, the pusher 64 may engage a distal bearing assembly 102b opposite a proximal bearing assembly 102a. In response to the axial force applied by the pusher 64 toward the distal gripping portion 40b of the collet 40, the distal bearing assembly 102b may be translated in the fore direction into a protruding foot of the closing collar 56. As shown, the closing collar may extend into a distal opening 82a formed by the bore 82 in the driveshaft 20, such that the distal bearing assembly 102 engages the exterior surface of the shaft wall 90 of the driveshaft 20 and further engages the protruding feet 112 or proximal protrusions formed by the closing collar 56. The closing collar 56 may correspond to a tubular body comprising the proximal protrusions 112 at a proximal end opposed by a tapered distal opening 114. The proximal protrusions 112 may align with and be received by distal receiving slots 118 formed in the distal end portion of the driveshaft assembly 50 and extending along the longitudinal axis A_{L}.

With the proximal protrusions 112 engaging the distal receiving slots 118, the translation of the pusher 64 may engage the distal bearing assembly 102b and further apply axial force to the proximal protrusions 112 of the closing collar 56. The axial force applied to the proximal protrusions 112 may result in the translation of the closing collar 56 distally along the longitudinal axis A_{L}. The axial travel T_{C} of the closing collar 56 may cause the tapered distal opening 114 to engage a distally expanding collet taper 116 formed by the clamping arms 84 of the collet 40. As a result of the tapered distal opening 114 engaging the distally expanding collet taper 116, the clamping arms 84 of the collet 40 may compress inward toward the collet axis A_{C}. Further, the collet axis A_{C} may be forced into alignment with the longitudinal axis A_{L} of the driveshaft assembly 50 and, more generally, of the driver assembly 10. In this way, the clamping of the collet 40 resulting from the engagement of the actuator assembly 30 may adjust the locking interface 36 into an engaged configuration wherein the collet axis A_{C} is aligned with the longitudinal axis A_{L}. The locking interface 36 between the collet assembly 34 and the driveshaft 20 may constrain and prevent the distal gripping portion 40b of the collet 40 from repositioning within the bore 82 when the pin or wire 42 is engaged by the collet assembly 34.

Referring now to FIGS. 5, 8A, and 8B, the driveshaft assembly 50 may further comprise a nose 120 that may extend outward from the distal collar 52b of the body assembly 52. The cannula 44 may extend centrally through a distal nose opening 122 formed through the nose 120 to receive or pass the pin or wire 42. Opposite the distal nose opening 122, a proximal fastening portion of the nose 120 may engage a distal fastening portion 126 formed by or in connection with a distal end of the driveshaft 20. In this configuration, the distal opening 82a of the bore 82 may be at least partially enclosed by the nose 120 and the nose 120 may be fastened into an assembled configuration with the driveshaft 20 by the engagement of the fastening portions 124, 126. The engagement of the nose 120 to the driveshaft 20 may further retain the components of the driveshaft assembly 50 including the underlying components of the collet assembly 34 between the axial positioning ring 106 located near the drive interface 18 of the driveshaft 20 and to the distal fastening portion 126 of the driveshaft 20. In this way, the driveshaft assembly 50 and collet assembly 34 may correspond to a modular subassembly that may be assembled and removed from the driver assembly 10 for repair or maintenance.

Referring again to FIG. 5, a variety of components forming the driveshaft assembly 50 and/or the actuator assembly 30 are described in reference to particular examples including various features that may be integrally formed as a single device or component. For example, the driveshaft 20 is described as having numerous features including the drive interface 18, the bore 82, distal receiving slots 118, and the distal fastening portion 126 implemented in a common, integral body. However, it shall be understood that these components or features may similarly be implemented by assembling multiple shaft components including tubular structures, fastening structures, shanks, and couplers, etc. Therefore, the features discussed in the application shall not be considered limiting to the scope of the claims. More generally, the components and devices discussed herein may provide for a variety of examples that may enable skilled persons to practice the inventive subject matter disclosed. Variations in the structures and interfaces, therefore, are considered within the scope of the disclosure.

According to some aspects of the disclosure, an actuator assembly for a collet of a surgical tool is provided. The collet operably connects to a driveshaft of a rotary tool, wherein the collet is selectively compressed responsive to a collar position of a closing collar. The actuator assembly comprises a lever that rotates about a lever axis and operatively engages the closing collar, thereby adjusting the collar position; a body comprising a support bracket and connecting an adjustable rotary assembly to the actuator assembly, wherein an indexing locator is selectively positioned rotationally about a pin axis defining a lever axis position of the lever axis; and a pivot pin interconnecting the lever to the body.

According to various aspects, the disclosure may implement one or more of the following features or configurations in various combinations:
- an index position of the indexing locator about the pin axis adjusts the lever axis position of the lever axis relative to the body;
- the indexing locator is positioned selectively between the pivot pin and the body in an index position of a plurality of predetermined rotary positions;
- the pivot pin comprises the indexing locator selectively positioned in the plurality of rotary positions of the body;
- the body comprises the indexing locator selectively positioning the pin in the plurality of rotary positions;
- the plurality of predetermined rotary positions are positioned about the pin axis along which the pin engages the body;
- the lever axis is eccentric to the pin axis;
- the lever axis is offset from the pin axis over an eccentric axis offset;
- an offset direction of the eccentric axis offset is adjusted based on an index position of the indexing locator about the pin axis;
- an index position of the indexing locator adjusts a lever angle in an unclamped position and a clamped position;
- relative position of a travel or throw of the lever from an unclamped position to a clamped position is adjusted based on the index position;
- the pin comprises a cylindrical fulcrum extending along the lever axis and about which the lever rotates;
- the pivot pin engages the body along the pin axis and the cylindrical fulcrum is offset from the pin axis;
- the pin axis extends along the pin from a proximal head to a distal threaded end portion;
- the indexing locator is positioned proximate to the head and the cylindrical fulcrum extends between the indexing locator and the distal threaded portion; and/or
- the indexing locator is angularly aligned with the offset of the lever axis relative to the pin axis.

According to another aspect of the disclosure, a method for adjusting an orientation of a lever arm of a collet assembly of a rotary surgical tool is provided. The method comprises determining a travel or throw of the lever arm of an actuator lever of the collet assembly relative to a handle of the rotary surgical tool in one of a clamped position and a released position; and adjusting an orientation of a pivot pin about a pin axis defining a lever axis about which the lever arm rotates, wherein the orientation of the pivot pin relative to the collet assembly defines an offset direction of the lever axis relative to the pin axis.

According to various aspects, the disclosure may implement one or more of the following features or configurations in various combinations:
- the adjusting the orientation of the pivot pin comprises positioning the pivot pin in one of a plurality of predefined rotary positions about the pin axis, thereby defining the offset direction of the lever axis; and/or
- disengaging the pivot pin from the lever arm and freely positioning a lever angle of the lever relative to the collet assembly and engaging the pivot pin in one of a plurality of predefined rotary positions about the pin axis defining the offset direction of the lever axis.

According to yet another aspect of the disclosure, an actuator for a surgical collet comprises a lever having a fulcrum comprising a load arm and an opposing force arm; a pivot pin comprising a shaft that engages the lever defining the fulcrum and extending between a head and a mating end portion along a pin axis, wherein the shaft defines a lever axis of the lever laterally offset over an offset distance from the pin axis; and a support body operably connected to the surgical collet, wherein the head and the mating end portion of the pivot pin engage the support body interconnecting the lever to the support body along the cylindrical lever fulcrum, wherein the pivot pin is positioned in one of a plurality of predefined orientations rotationally about the pin axis defining an offset direction of the lever axis relative to the body.

According to various aspects, the disclosure may implement one or more of the following features or configurations in various combinations:
- the load arm engages a pusher assembly that translates along a longitudinal axis of the collet and selectively clamps and releases the collet based on a lever angle of the lever;
- the pivot pin comprises at least one locating feature that operably couples the pivot pin to the support body in one of the plurality of predefined orientations rotationally about the pin axis; and/or
- the at least one locating feature comprises a protrusion forming an indexing locator that engages one of a plurality of receiving openings defining the plurality of predefined orientations.

According to some aspects of the disclosure, a wire or pin driver for a rotary surgical tool comprises a driveshaft having a comprising a bore and extending along a longitudinal axis; a collet forming a cannula extending from a proximal engagement portion disposed in the bore to a distal gripping end configured to engage an elongated shaft (a wire, pin, etc.); and at least one retaining insert extending between and engaging the proximal engagement portion and the driveshaft in the bore.

According to various aspects, the disclosure may implement one or more of the following features or configurations in various combinations:
- at least one retaining insert engages the collet and the driveshaft over a clearance distance between the collet and the driveshaft in the bore forming a rotary coupling;
- the distal gripping end of the collet is free to pivot within the bore about a pivot point defined by the rotary coupling;
- at least one retaining insert comprises a plurality of retaining inserts angularly distributed about the longitudinal axis between the collet and the driveshaft;
- the driveshaft comprises at least one receiving aperture that receives the retaining insert;
- the proximal engagement portion of the collet comprises at least one pocket aligned with the at least one receiving aperture by the engagement with the retaining insert;
- a retaining ring formed about the driveshaft, wherein the retaining ring encloses the retaining insert in the at least one receiving aperture and in alignment with the at least one pocket;
- the retaining ring forms a portion of a proximal thrust bearing assembly enclosed about the driveshaft and the bore;
- an actuator assembly that at least partially encloses the collet and selectively compresses the distal gripping end;
- the actuator assembly comprises a proximal thrust bearing assembly enclosed about the at least one aperture of the driveshaft and retaining the retaining insert engaged with the collet and the driveshaft;
- the actuator assembly comprises a closer configured to engage the collet and interposed in the bore between the driveshaft and the collet, wherein the closer applies radial pressure to the distal gripping end in response to actuation of a lever of the actuator assembly;
- the actuator assembly comprises a pusher operatively coupled to the adjustment lever, wherein the pusher is selectively positioned along the longitudinal axis in response to an orientation of a positioning lever relative to the driveshaft, and wherein the position of the pusher along the longitudinal axis causes the closer to apply and release the radial pressure of the distal gripping end;
- the actuator assembly comprises a closer comprising a hollow, tube-like body enclosed about the collet in the bore inside a perimeter wall of the driveshaft, wherein the closer comprises a plurality of proximal protrusions that extend through the perimeter wall within elongated slots formed in a distal end portion of the driveshaft; and/or
- the proximal protrusions are engaged by a pusher that changes a position of the closer or closing collar along the longitudinal axis in response to an actuation of a lever of the actuator assembly.

According to another aspect of the disclosure, a collet assembly for a surgical pin driver comprises a collet extending along a collet axis from a proximal engagement portion to a distal gripping end configured to engage a wire or pin, wherein the engagement portion forms an exterior mating surface comprising a plurality of exterior openings angularly spaced about the longitudinal axis; and a rotary shaft extending from a proximal drive interface to a collet interface comprising a bore extending along a drive axis, wherein the bore forms an interior mating surface comprising a plurality of interior openings formed in the collet interface, wherein the plurality of exterior openings and the plurality of interior openings are aligned angularly spaced about the collet axis and the drive axis and receive a plurality of retaining inserts rotationally binding the collet to the rotary shaft about the longitudinal axis.

According to various aspects, the disclosure may implement one or more of the following features or configurations in various combinations:
- the exterior openings are pockets formed in the exterior mating surface and the interior openings are apertures extending through the wall of the shaft along the collet interface;
- the exterior openings are spherical pockets and the retaining inserts are balls received in the apertures and the spherical pockets;
- the interior mating surface forms an inside diameter spaced from an outside diameter of the exterior mating surface over a first clearance distance, and a second clearance distance is formed between the retaining inserts and the interior openings of the shaft; and/or
- at least one of the first clearance distance and the second clearance distance form a clearance space allowing the collet axis to diverge from the drive axis at the distal gripping end.

According to yet another aspect of the disclosure, a wire driver for a rotary surgical tool comprises a driveshaft extending along a longitudinal axis and comprising a bore having an interior wall forming a collet interface and a plurality of receiving apertures formed through the collet interface. A collet forms a cannula extending from a proximal engagement portion disposed along the collet interface in the bore to a distal gripping end configured to engage a wire or pin, wherein the proximal engagement portion comprises a plurality of receiving pockets angularly aligned with the receiving apertures. A retaining insert extends through each of the receiving apertures and into the receiving pockets; and a retaining ring extending about the receiving openings formed in the collet interface of the driveshaft, wherein the retaining ring constrains the retaining insert within the receiving apertures and binds the retaining apertures generally aligned with the receiving pockets.

According to various aspects, the disclosure may implement the following features or configurations in various combinations:
- the retaining ring is formed by a portion of a bearing assembly enclosed within the driveshaft assembly.

It will be understood that any described processes or steps within described processes may be combined with other disclosed processes or steps to form structures within the scope of the present device. The exemplary structures and processes disclosed herein are for illustrative purposes and are not to be construed as limiting.

It is also to be understood that variations and modifications can be made on the aforementioned structures and methods without departing from the concepts of the present device, and further it is to be understood that such concepts are intended to be covered by the following claims unless these claims by their language expressly state otherwise.

The above description is considered that of the illustrated embodiments only. Modifications of the device will occur to those skilled in the art and to those who make or use the device. Therefore, it is understood that the embodiments shown in the drawings and described above are merely for illustrative purposes and not intended to limit the scope of the device, which is defined by the following claims as interpreted according to the principles of patent law, including the Doctrine of Equivalents

## Claims

1. An actuator assembly (30) for a collet (40) of a surgical tool, the collet (40) operably connected to a driveshaft of a rotary tool, wherein the collet (40) is selectively compressed responsive to a collar position of a closing collar (56), the actuator assembly (30) comprising:
a lever (32) that rotates about a lever axis (A_{LEV}) and operatively engages the closing collar (56), thereby adjusting the collar position;
a body comprising a support bracket (62) and connecting an adjustable rotary assembly to the actuator assembly (30), wherein an indexing locator (60a) is selectively positioned rotationally about a pin axis (A_{P}) defining a lever axis position of the lever axis (A_{LEV}); and
a pivot pin (60) interconnecting the lever (32) to the body.

2. The actuator assembly (30) according to claim 1, wherein an index position of the indexing locator (60a) about the pin axis (A_{P}) adjusts the lever axis position of the lever axis (A_{LEV}) relative to the body.

3. The actuator assembly (30) according to any one of claims 1-2, wherein the indexing locator (60a) is positioned selectively between the pivot pin (60) and the body in an index position of a plurality of predetermined rotary positions (74).

4. The actuator assembly (30) according to claim 3, wherein the pivot pin (60) comprises the indexing locator (60a) selectively positioned in the plurality of rotary positions (74) of the body.

5. The actuator assembly (30) according to any one of claims 3-4, wherein the body comprises the indexing locator (60a) selectively positioning the pin in the plurality of rotary positions (74).

6. The actuator assembly (30) according to any one of claims 3-5, wherein the plurality of predetermined rotary positions (74) are positioned about the pin axis (A_{P}) along which the pin engages the body.

7. The actuator assembly (30) according to any one of claims 1-6, wherein the lever axis (A_{LEV}) is eccentric to the pin axis (A_{P}).

8. The actuator assembly (30) according to claim 7, wherein the lever axis (A_{LEV}) is offset from the pin axis (A_{P}) over an eccentric axis offset.

9. The actuator assembly (30) according to claim 8, wherein an offset direction of the eccentric axis offset is adjusted based on an index position of the indexing locator (60a) about the pin axis (A_{P}).

10. The actuator assembly (30) according to any one of claims 1-9, wherein an index position of the indexing locator (60a) adjusts a lever angle (θ) in an unclamped position and a clamped position.

11. The actuator assembly (30) according to any one of claims 1-10, wherein a relative position of a travel or throw of the lever (32) from an unclamped position to a clamped position is adjusted based on the index position.

12. The actuator assembly (30) according to any one of claims 1-11, wherein the pin comprises a cylindrical fulcrum extending along the lever axis (A_{LEV}) and about which the lever (32) rotates.

13. The actuator assembly (30) according to claim 12, wherein the pivot pin (60) engages the body along the pin axis (A_{P}) and the cylindrical fulcrum is offset from the pin axis (A_{P}).

14. The actuator assembly (30) according to claim 13, wherein the pin axis (A_{P}) extends along the pin from a proximal head (60c) to a distal threaded end portion.

15. The actuator assembly (30) according to claim 14, wherein the indexing locator (60a) is positioned proximate to the head and the cylindrical fulcrum extends between the indexing locator (60a) and the distal threaded portion.
